# EUROPÄISCHE PATENTSCHRIFT

(11) **EP 2 467 103 B1**
(45) Veröffentlichungstag und Bekanntmachung des Hinweises auf die Patenterteilung: **28.08.2013**
(21) Anmeldenummer: 10751785.6
(22) Anmeldetag: 20.07.2010
(51) Int. Cl.: B25J 9/10, A61F 2/58, B25J 15/00

(54) **GREIFEINRICHTUNG**
GRIPPING ASSEMBLY
DISPOSITIF DE PRÉHENSION

(30) Priorität: 19.08.2009 DE 102009037898
(43) Veröffentlichungstag der Anmeldung: 27.06.2012
(73) Patentinhaber: Otto Bock Healthcare Products GmbH, 1070 Wien (AT)
(72) Erfinder: HASLINGER, Martin, A-3653 Weiten (AT)
(74) Vertreter: Stornebel, Kai
(86) Internationale Anmeldenummer: PCT/EP2010/004419
(87) Internationale Veröffentlichungsnummer: WO 2011/020535

(56) Entgegenhaltungen:
- WO-A1-03/080297
- WO-A1-2008/092695
- DE-A1-102007 022 973
- YOUNG JUNE SHIN ET AL: "Application of sliding actuation mechanism to robot finger" ADVANCED INTELLIGENT MECHATRONICS, 2009. AIM 2009. IEEE/ASME INTERNATIONAL CONFERENCE ON, IEEE, PISCATAWAY, NJ, USA, 14. Juli 2009 (2009-07-14), Seiten 550-553, XP031524154 ISBN: 978-1-4244-2852-6

## Beschreibung

Die Erfindung betrifft eine Greifeinrichtung mit einem Proximalglied, einem Medialglied und einem Distalglied, die verschwenkbar aneinander gelagert sind, und mit einem Aktuator der mit einem verschieblich gelagerten Kopplungselement gekoppelt ist, das Kopplungselement ist zwischen dem Proximalglied und dem Distalglied angeordnet und mit dem Proximalglied und dem Distalglied kraftübertragend verbunden.

Eine solche Greifeinrichtung ist insbesondere für Handprothesen sinnvoll, bei denen die Greifeinrichtung als ein Prothesenfinger eingesetzt werden kann. Neben prothetischen Einsatzgebieten ist es möglich, die Greifeinrichtung auch auf anderen Technikgebieten einzusetzen, beispielsweise in der Handhabungstechnik oder bei sogenannten Robotern.

Aus der DE 10 2007 005 858 A1 ist eine gattungsgemäße Greifeinrichtung in Ausgestaltung eines Prothesenfingers bekannt. Dabei ist in dem Medialglied ein längsbeweglicher Waagebalken angeordnet, der über Hebel mit dem Proximalglied und dem Distalglied verbunden ist. Die Anbindung des Waagebalkens über Hebel an das Proximalglied und das Distalglied ist mechanisch aufwendig. Darüber hinaus ist es notwendig, das Medialglied mit den Hebeln zu kapseln, um einen einsatzbereiten Finger herzustellen.

Aufgabe der vorliegenden Erfindung ist es, eine Greifeinrichtung bereitzustellen, die robuster ausgebildet und einfacher herzustellen ist.

Erfindungsgemäß wird diese Aufgabe durch eine Greifeinrichtung mit den Merkmalen des Hauptanspruches ausgelöst. Vorteilhafte Ausgestaltungen und Weiterbildungen der Erfindung sind in den Unteransprüchen ausgeführt.

Die erfindungsgemäße Greifeinrichtung mit einem Proximalglied, einem Medialglied und einem Distalglied, die jeweils verschwenkbar aneinander gelagert sind, und mit einem Aktuator, der, ggf. über eine Getriebeeinrichtung, mit einem verschieblich gelagerten Kopplungselement gekoppelt ist, wobei das Kopplungselement zwischen dem Proximalglied und dem Distalglied angeordnet ist und sowohl mit dem Proximalglied als auch mit dem Distalglied kraftübertragend verbunden ist, sieht vor, dass an dem Kopplungselement zumindest ein Hebel angeordnet ist, der sowohl mit dem Proximalglied als auch mit dem Distalglied verbunden ist und das Proximalglied mit dem Distalglied kinematisch koppelt. Durch die Integration der Kraftübertragung in einen Hebel ist es möglich, dass die einzelnen Glieder auf eine einfache Art und Weise unmittelbar miteinander gekoppelt werden, so dass eine abgestimmte Greifbewegung durch das Verfahren des Kopplungsgliedes erreicht werden kann. Dies bedeutet eine Verringerung der Teileanzahl und eine Stabilisierung der mechanischen Kraftübertragungskette, so dass neben einer ökonomischen Verbesserung auch eine mechanische Verbesserung aufgrund einer verbesserten Stabilität innerhalb der Greifeinrichtung gewährleistet werden kann. Darüber hinaus kann der Hebel so ausgebildet sein, dass er das gesamte Medialglied überdeckt, dass keine zusätzliche Umhüllung zum Schutz der Mechanik notwendig ist. Es ist nur noch ein Hebel für die Kopplung des Kopplungselementes mit dem Proximalglied und dem Distalglied notwendig.

Der Aktuator, der insbesondere als Motor aber auch als Zugmittelantrieb, angetriebene Welle, Kardanantrieb oder dergleichen ausgebildet sein kann, kann das Kopplungselement antreiben, um so die Verlagerung des Kopplungselementes zu bewirken. Zwischen dem Aktuator und dem Kopplungselement kann eine Getriebeeinrichtung angeordnet sein, die Kegel- und/oder Kronenräder oder andere Zahnräder, Rollen oder dergleichen aufweisen kann, um die Bewegung des Akuators auf das Kopplungselement zu übertragen. Eine Spindel kann Teil der Getriebeeinrichtung sein, ebenso können Wellen, Zugmitteltriebe, Gelenke oder andere Getriebeelemente eingesetzt werden, um die Bewegung umzulenken, zu verlangsamen oder zu beschleunigen und an den gewünschten Ort zu bringen. Statt einer Getriebeeinrichtung kann ein Seilzug, ein passiver Antrieb, eine biegsame Welle oder ein kardanischer Antrieb vorgesehen sein, so dass auf ein Getriebe verzichtet werden kann.

Vorteilhafterweise ist das Kopplungselement als eine Spindelmutter ausgebildet, so dass durch eine rotatorische Bewegung einer Spindel, die in dem Medialglied gelagert ist, eine Längsverschieblichkeit des Kopplungselementes gewährleistet werden kann. Dadurch ist es auf einfache Art und Weise möglich, sowohl das Medialglied relativ zu dem Proximalglied als auch das Distalglied relativ zu dem Medialglied durch eine einzige rotatorische Antriebsbewegung zu verlagern.

Das Kopplungselement, z.B. die Spindelmutter, kann drehstarr mit dem Hebel gekoppelt sein, so dass der Hebel ausschließlich eine translatorische Bewegung ausführt.

An dem Proximalglied ebenso wie an dem Distalglied sind Anlenkpunkte für den Hebel vorgesehen, die auf einander gegenüberliegenden Seiten einer Verbindungsebene liegen, die durch die Schwenkachsen des Medialgliedes und des Distalgliedes verläuft. Da sämtliche Glieder der Greifeinrichtung schwenkbar miteinander verbunden sind und die Schwenkachsen vorzugsweise parallel zueinander angeordnet sind, ist es möglich, eine Ebene durch die Schwenkachse des Medialglieds und des Distalgliedes zu legen. Das Distalglied ist auf der einen Seite dieser Ebene an dem Hebel angelenkt und das Proximalglied auf der gegenüberliegenden Seite der Ebene. Dadurch kann durch eine einfache translatorische Bewegung eine Greifverrichtung ausgeführt werden, die der Bewegung eines Fingers entspricht. Dabei sind die Bewegungen zwischen dem Proximalglied und dem Medialglied und zwischen dem Medialglied und dem Distalglied kinematisch fest miteinander gekoppelt. Welche Bewegungsumfänge, also welche Drehwinkel, sich bei einer gegebenen Verschiebung des Hebels ergibt, hängt von der Geometrie der Anlenkpunkte ab. Durch eine Verlagerung der Anlenkpunkte ist es möglich, die Übersetzung des Hebelgetriebes zu verändern, so dass auch unterschiedliche Verschwenkwinkel des Distalgliedes und des Medialgliedes erreicht werden können.

Bevorzugt ist der Hebel einstückig ausgebildet, um eine ausreichende mechanische Stabilität und eine einfache Herstellbarkeit zu gewährleisten. Sind beidseitig entlang der Verschiebeachse des Kopplungselementes Hebel angeordnet, kann eine besonders symmetrische Kraftübertragung gewährleistet werden, so dass eine Verkantung der Glieder zueinander ausgeschlossen werden kann. Dabei sind die bevorzugt einstückigen Hebel an dem Medialglied angeordnet und bilden die äußere Struktur des Medialgliedes. Alternativ oder ergänzend ist vorgesehen, dass ein elastisches Zusatzelement zwischen dem Hebel und dem Kopplungselement angeordnet ist, um eine verbesserte Nachgiebigkeit zu erreichen. Das Zusatzelement kann an beiden Enden des Hebels angeordnet sein. Weiterhin ist alternativ oder ergänzend vorgesehen, dass ein Zusatzelement zwischen dem Hebel und dem Distalglied und/oder dem Proximalglied angeordnet ist, um die Nachgiebigkeit zu erhöhen. Das Zusatzelement kann als separates Bauteil ausgebildet sein oder an den Hebel, das Kopplungselement und/oder dem Distal- und/oder Proximalglied angeformt sein.

Der Aktuator, z.B. Motor, ist bevorzugt in dem Proximalglied angeordnet, da in dem Proximalglied in der Regel der meiste Platz ist und über eine Getriebeeinrichtung, beispielsweise ein Kegel- oder Kronenradgetriebe, ein Seilantrieb, ein Kardangelenk, eine biegsame Welle oder andere Getriebeelemente, eine Drehbewegung sehr leicht auf eine Spindel übertragen werden kann. Auch ist es möglich, über eine Kardankopplung die rotatorische Bewegung eines Aktuators, z.B. eines Motors in das Medialglied zu übertragen, um eine translatorische Bewegung des Kopplungselementes zu bewirken. Die Anordnung des Motors in dem Proximalglied hat den Vorteil, dass die Energieversorgung sehr leicht zugeführt werden kann, so dass die Medial- und Distalglieder lediglich mechanische Komponenten enthalten, die zur Greifverrichtung ausgebildet sind. Dementsprechend können die Medial- und Distalglieder leichter ausgebildet werden, wodurch der Motor selbst kleiner ausgelegt werden kann. Es kann auch vorgesehen sein, dass der Aktuator in dem Distalglied angeordnet ist, was den Vorteil hat, dass elektronische Elemente und/oder stromgebende Elemente in dem Proximalglied untergebracht werden können, was im Hinblick auf einen Teilhandersatz sinnvoll erscheint und auch bei anderen Anwendungsgebieten der Greifeinrichtung jenseits der Prothetik realisierbar ist.

Nachfolgend wird ein Ausführungsbeispiel der Erfindung anhand der beigefügten Figuren näher erläutert. Es zeigen:
- Figur 1 -: eine Seitenansicht einer gestreckten Greifeinrichtung,
- Figur 2 -: eine Seitenansicht einer leicht gekrümmten Greifeinrichtung;
- Figur 3 -: eine Seitenansicht einer vollständig gekrümmten Greifeinrichtung;
- Figur 4 -: eine Explosionszeichnung einer gestreckten Greifeinrichtung;
- Figur 5 -: eine teilweise zusammengebaute Greifeinrichtung;
- Figur 6 -: eine Untenansicht einer Greifeinrichtung;
- Figur 7 -: eine Draufsicht einer Greifeinrichtung;
- Figur 8 -: eine Detaildarstellung des Antriebs mit Kraftübertragungseinrichtungen,
- Figur 9 -: eine Detaildarstellung eines Medialgliedes;
- Figur 10 -: eine Draufsicht auf ein Medialglied;
- Figur 11 -: eine Detaildarstellung einer Spindel und einer Spindelmutter;
- Figur 12 -: eine Detailansicht einer Spindelmutter;
- Figur 13 -: eine Querschnittsansicht durch die Antriebseinrichtung; sowie
- Figur 14 -: eine Detaildarstellung eines Reibradgetriebes;

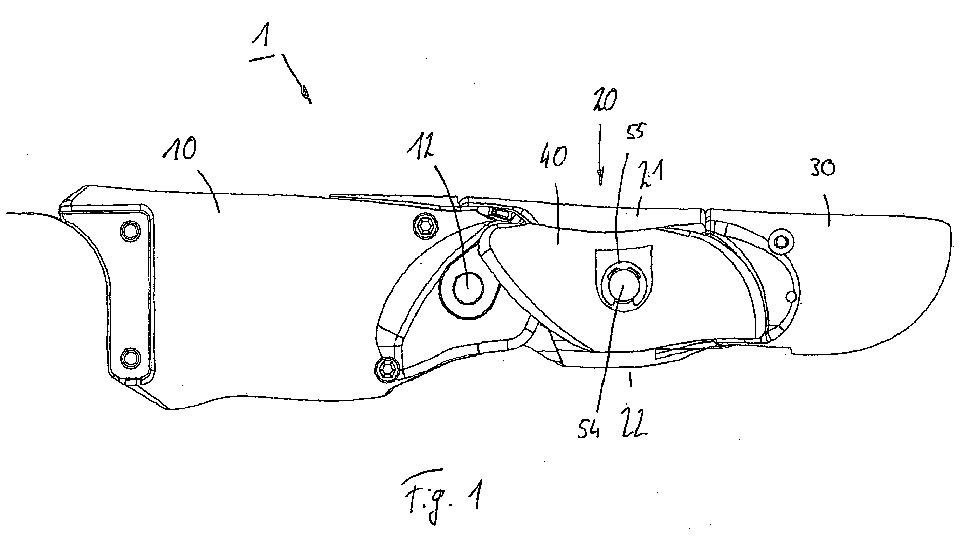

In der Figur 1 ist in einer Seitenansicht eine Greifeinrichtung 1 in Gestalt eines Prothesenfingers mit einem Proximalglied 10, einem Medialglied 20 und einem Distalglied 30 dargestellt. Das Proximalglied 10 kann an einem nicht dargestellten Chassis befestigt sein, dabei kann es entweder fest daran angeordnet oder über einen Antrieb gelenkig angetrieben sein, so dass sich das Proximalglied 10 relativ zu dem nicht dargestellten Chassis verlagern lässt, ähnlich einem Fingergrundgelenk.

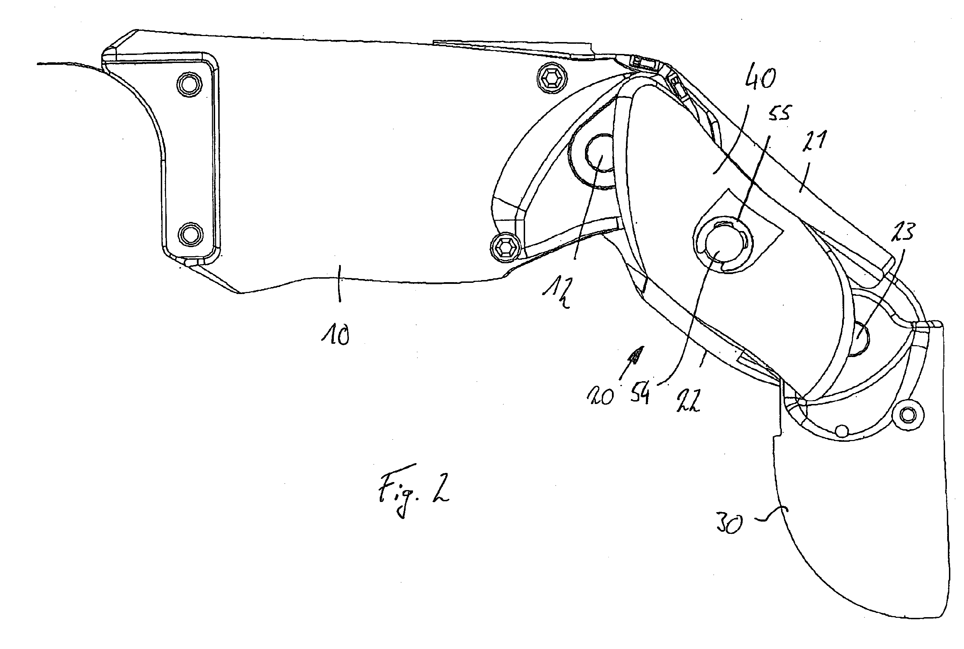

An dem distalen Ende des Proximalgliedes 10 ist das Medialglied 20 schwenkbar um eine Schwenkachse 12 gelenkig gelagert. An dem Medialglied 20 ist an dem distalen Ende das Distalglied 30 gelenkig gelagert. Die Schwenkachse 23, um die das Distalglied 30 verschwenkt, ist in der Figur 2 zu sehen.

In der Figur 1 ist die Greifeinrichtung 1 in einer gestreckten Stellung dargestellt. An dem Medialglied 20 sind seitlich Hebel 40 angeordnet, wobei in der Seitenansicht nur ein Hebel 40 zu sehen ist. Eine obere Abdeckung 21 und eine untere Abdeckung 22 sind auf der Oberseite und Unterseite des Medialgliedes 20 angeordnet. In dem Medialglied 20 ist eine längsverschiebliche Kopplungseinrichtung angeordnet, von der nur die Achse 54 zu sehen ist. Auf der Achse 54 ist der Hebel 40 über eine Federscheibe 55 gehalten. Die Hebel 40 sind kraftübertragend und relativ zu dem Proximalglied 10 und dem Distalglied 30 verschwenkbar an dem Proximalglied 10 und dem Distalglied 30 kraftübertragend gelagert. Die Lagerungsart und die Lagerungsanordnung wird später erläutert werden.

In der dargestellten Ausführungsform in Figur 1 ist das Kopplungselement gemeinsam mit den Hebeln 40 in der distalen Stellung gezeigt, also in derjenigen Stellung, in der die Achse 54 am weitesten in Richtung auf das Distalglied 30 verfahren ist.

In der Figur 2 ist die Greifeinrichtung 1 gemäß Figur 1 in einer leicht gebeugten Stellung dargestellt. Das Kopplungselement mit der gezeigten Achse 54 ist in eine mittlere Position verfahren, wodurch aufgrund der diagonal zu einer Ebene zwischen den Schwenkachsen 12, 23 angeordneten Anlenkpunkte der Hebel 40, das Medialglied 20 um ca. 45° nach unten verschwenkt wurde. Ebenfalls wurde das Distalglied 30 um ungefähr 45° zu dem Medialglied 20 verschwenkt, wodurch insgesamt das Distalglied 30 um ungefähr 90° relativ zu dem Proximalglied 10 verlagert wurde. Die Kraftübertragung von dem Kopplungselement auf das Proximalglied 10 und das Distalglied 30 erfolgt unmittelbar durch den einstückigen Hebel 40, der die Glieder direkt miteinander koppelt.

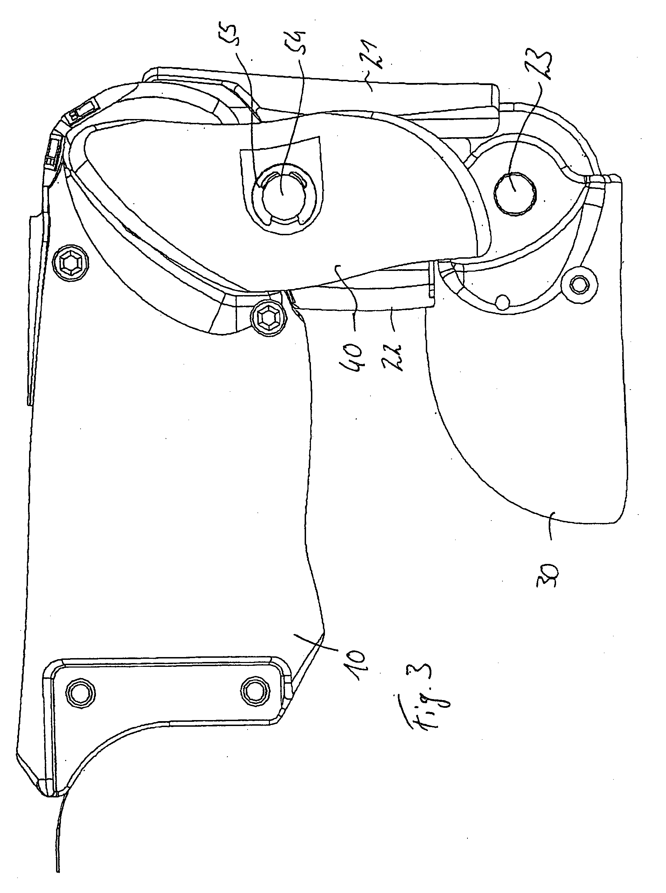

In der Figur 3 ist die maximal gebeugte Stellung des Distalgliedes 30 gezeigt. Die Achse 54 des Kopplungselementes ist dabei maximal an das Proximalglied 10 herangefahren, das Medialglied 20 steht ungefähr in einem 90° Winkel zu dem Proximalglied 10, das Distalglied 30 steht in einem 90° Winkel zu dem Medialglied 20. Die Stellung gemäß der Figur 3 entspricht einem maximal gekrümmten Finger mit zwei Fingergelenken. Die Verlagerungsbewegung des Hebels 40 zwischen den Stellungen gemäß der Figur 1 und der Figur 3 kann daran erkannt werden, dass in der Figur 1 die Schwenkachse 12 des Medialglieds relativ zu dem Proximalglied 10 zu erkennen ist, während die ursprünglich abgedeckte Schwenkachse 23 des Distalgliedes 30 relativ zu dem Medialglied 20 in der Figur 3 sichtbar ist.

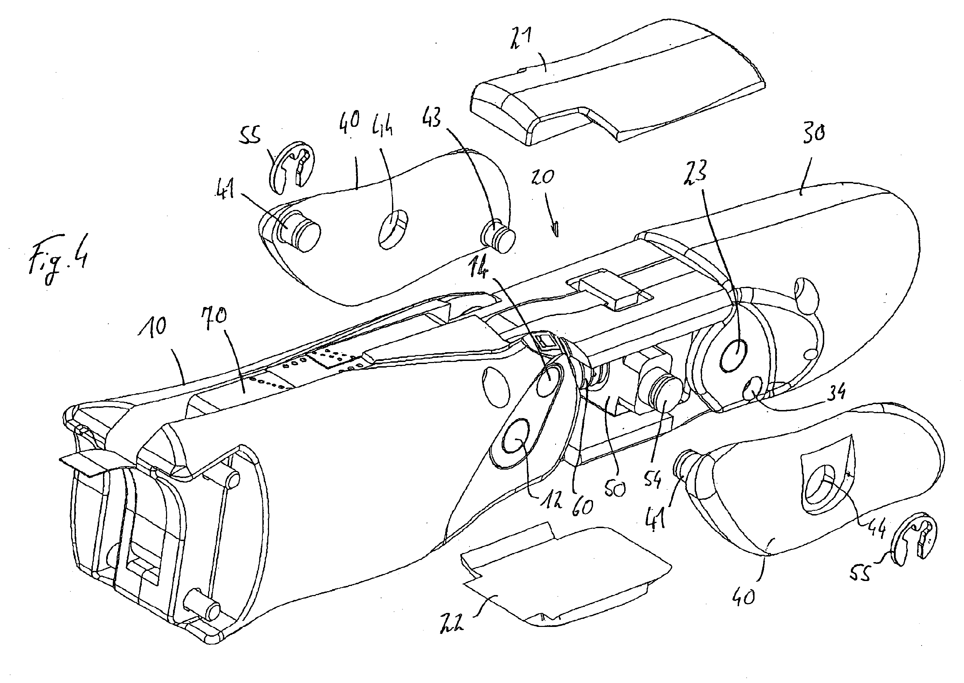

In der Figur 4 ist der Grundaufbau eines Greifelementes 1 in Gestalt einer Explosionsdarstellung gezeigt. Innerhalb des Proximalgliedes 10 ist ein Antrieb 70 integriert, der später näher erläutert werden wird. Oberhalb der Schwenkachse 12 sind beiderseits der Längserstreckung, also an den Seitenflächen des Proximalglieds 10, Aufnahmebohrungen 14 angeordnet, in die Zapfen 41 der Hebel 40 eingreifen. Unterhalb der Ebene zwischen den beiden Schwenkachsen 12, 23 ist am Distalglied 30 eine entsprechende Aufnahmebohrung 34 ausgebildet, die zur Aufnahme des Zapfens 43 dient. Innerhalb der Hebel 40 sind Bohrungen 44 ausgebildet, durch die die Achse 54 des Kopplungselementes 50 hindurchgeht. Zur Montage der Hebel 40 müssen die Zapfen 41, 43 in die Aufnahmebohrungen 14, 34 eingeführt und die Achse 54 durch die Bohrung 44 in dem Hebel 40 durchgesteckt werden. Eine Sicherung der Hebel 40 an der Achse 54 erfolgt über die Federscheiben 55, die von oben aufgesteckt werden.

Innerhalb des Medialgliedes 20 ist eine Antriebsspindel 60 angeordnet, die von der Antriebseinheit 70 angetrieben wird. Die Antriebsspindel 60 ist drehbar und unverschieblich in dem Medialglied 20 gelagert. Auf der Antriebsspindel 60 ist das Kopplungselement 50 gelagert, wobei das Kopplungselement 50 gegen eine Rotationsbewegung gesichert ist, so dass bei Drehung der Antriebsspindel 60 die Rotationsbewegung der Spindel 60 in eine Translationsbewegung des Kopplungselementes 50, das als Spindelmutter ausgebildet ist, umgewandelt wird. Wird die Spindel 60 angetrieben, wird das Kopplungselement 50 je nach Drehrichtung in Richtung auf das Proximalglied 10 oder das Distalglied 30 verlagert. Bei einer Verlagerung in Richtung auf das Distalglied 30 wird bei der vorliegenden kinematischen Anordnung eine Streckung des Medialgliedes 20 und des Distalgliedes 30 bewirkt, die in der Position der gestreckten Stellung gemäß Figur 1 endet. Bei einer umgekehrten Drehbewegung werden das Medialglied 20 und das Distalglied 30 um die Schwenkachsen 12, 23 verschwenkt, bis die Endstellung gemäß Figur 3 erreicht ist.

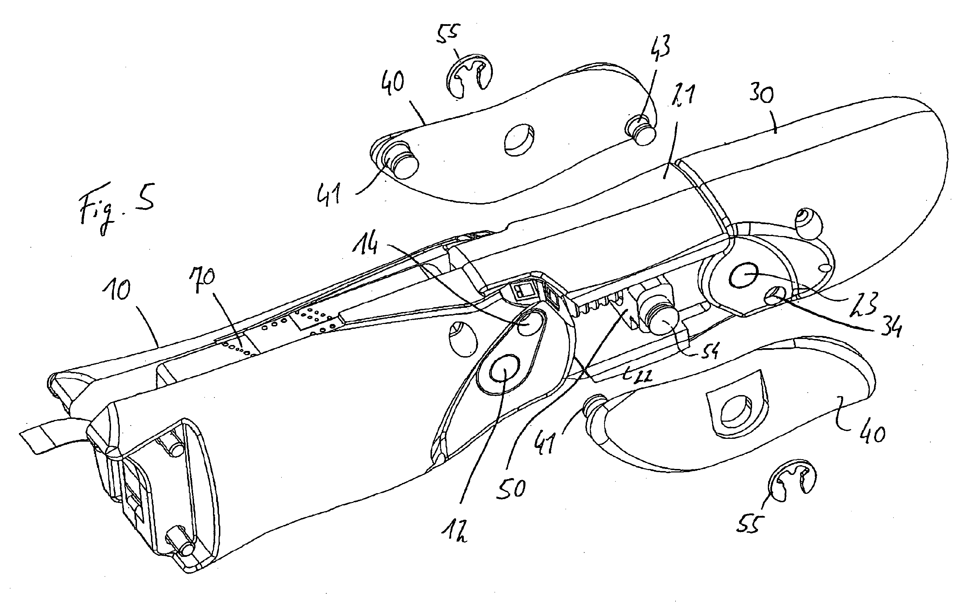

In der Figur 5 ist die Greifeinrichtung 1 gemäß Figur 4 in einem teilmontierten Zustand gezeigt, die oberen und unteren Abdeckungen 21, 22 sind bereits montiert, die seitlichen Hebel 40 sind noch nicht an dem Kopplungselement 50 und dem Distalglied 30 und dem Proximalglied 10 befestigt. Bei einer anderen Anordnung der Aufnahmebohrungen 14, 34 würde sich bei einer Bewegung des Kopplungselementes 50 in Richtung auf das Distalglied 30 eine Krümmung, also eine Biegung des Distalgliedes 30 relativ zu dem Medialglied 20 und des Medialgliedes 20 relativ zum dem Proximalglied 10 nach unten ergeben, bei einer Bewegung in Richtung auf das Proximalglied 10 eine Streckung.

Statt der dargestellten gleichmäßigen Beugung, bei der das Medialglied 20 um den gleichen Winkel relativ zu dem Proximalglied 10 wie das Distalglied 30 zu dem Medialglied 20 verschwenkt wird, kann durch eine entsprechende Anordnung der Aufnahmebohrungen 14, 34 auch eine Übersetzungsveränderung erfolgen, so dass bei der Verschiebung der Hebel 40 unterschiedliche Verschwenkwinkel der einzelnen Glieder realisiert werden können.

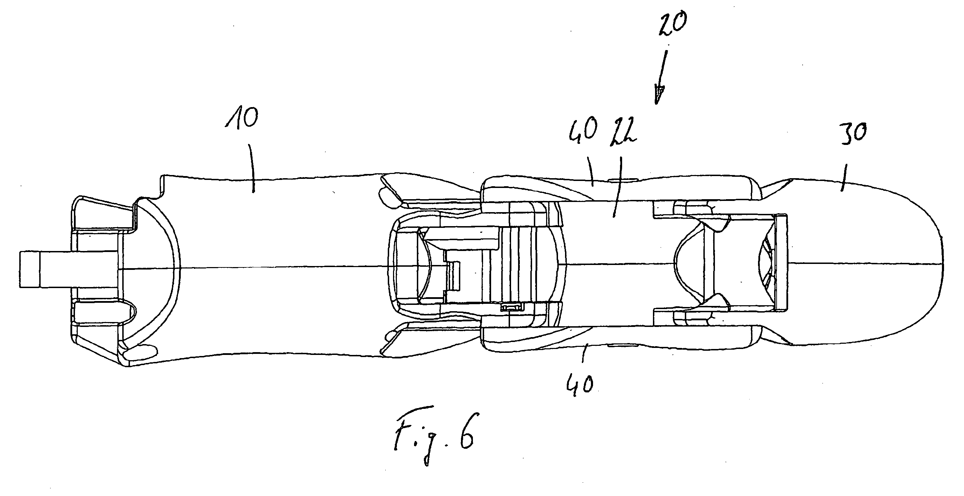

In der Figur 6 ist in einer Untenansicht die Greifeinrichtung 1 mit den einzelnen Gliedern 10, 20, 30 dargestellt. Die beiden Hebel 40 sind beidseitig entlang der Längserstreckung der Greifeinrichtung an dem Medialglied 20 montiert, die untere Abdeckung 22 ist ebenfalls angeordnet, um die Antriebsmechanik mit der Spindel 60 und dem Kopplungselement 50 zu schützen. Das Kopplungselement 50 und die Antriebsspindel 60 sind als Bewegungsgetriebe ausgebildet, wobei eine Drehung des Kopplungselementes 50 vorwiegend durch die Festlegung der Achsen 54 in den Durchgangslöchern 44 der Hebel 40 verhindert wird, da die Hebel 40 über die Zapfen 41, 43 drehfest mit dem Proximalglied 10 und dem Distalglied 30 gekoppelt sind.

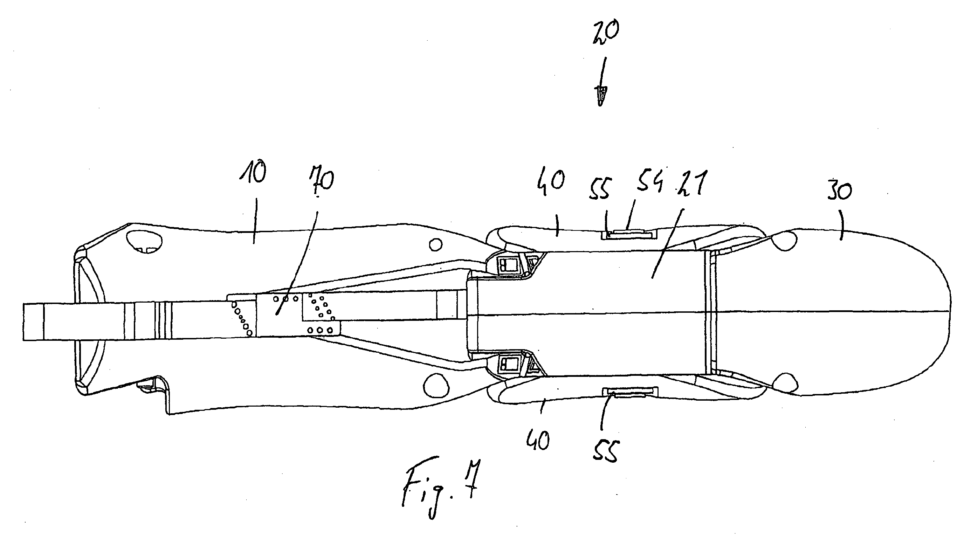

In der Figur 7 ist die Greifeinrichtung in Draufsicht gezeigt. Hier ist die Fixierung der Hebel 40 an den Achsen 54 über die Federscheiben 55 deutlich zu erkennen. Auch ist zu erkennen, dass das Proximalglied 10 und das Distalglied 30 in Längserstreckung geteilt ausgebildet sind, um eine leichtere Montierbarkeit zu ermöglichen.

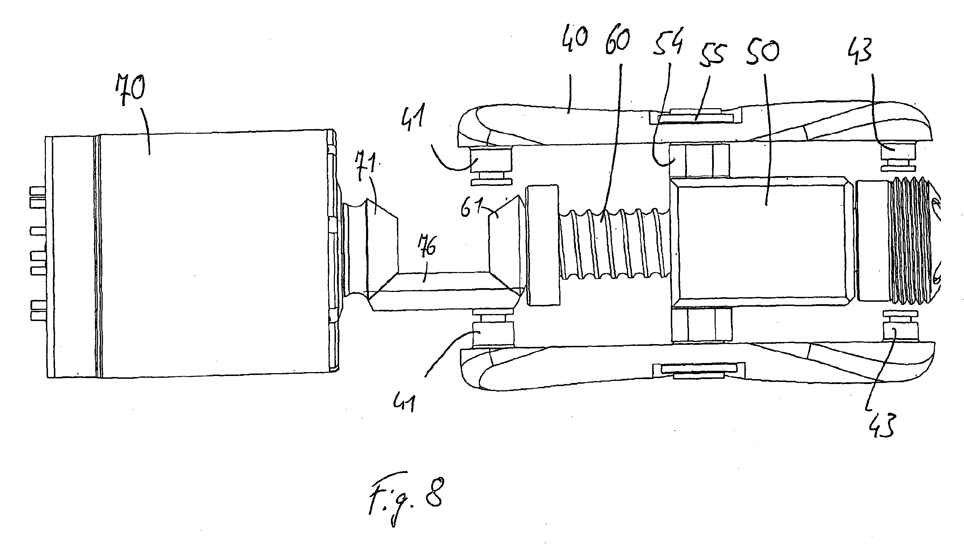

In der Figur 8 sind die Komponenten dargestellt, die zur Bewegung der Glieder der Greifeinrichtung 1 benötigt werden. Die Antriebseinheit 70 weist einen nicht näher dargestellten Aktuator in Gestalt eines Motors und ein Getriebe auf, an dessen Abtriebswelle ein Antriebszahnrad 71 angeordnet ist, das als Kegelrad ausgebildet ist. Das Antriebskegelrad 71 wird entsprechend der Drehrichtung des Motors in die eine oder andere Richtung verdreht und überträgt das Antriebsmoment über ein Übertragungszahnrad 76, das konzentrisch um die Drehachse 12 gelagert ist, auf das die Antriebsspindel 60 zugeordnete Kegelrad 61, das drehfest mit der Spindel 60 gekoppelt ist. Die Spindel 60 ist drehbar und unverschieblich gelagert, so dass bei Drehung der Spindel 60 das darauf angeordnete Kopplungselement 50 in Richtung auf das Kegelrad 61 verschoben oder davon wegbewegt wird. Je nach Verschieberichtung des Kopplungselementes 50 werden dann die beiden Hebel 40, die über die Achsen 54 mit dem Kopplungselement 50 verbunden sind, und damit auch die Zapfen 41, 43 in die ein oder andere Richtung verlagert und bewirken dadurch eine Verschwenkbewegung des Medialglieds 20 zu dem Proximalglied 10 und gleichzeitig des Distalgliedes 30 zu dem Medialglied 20.

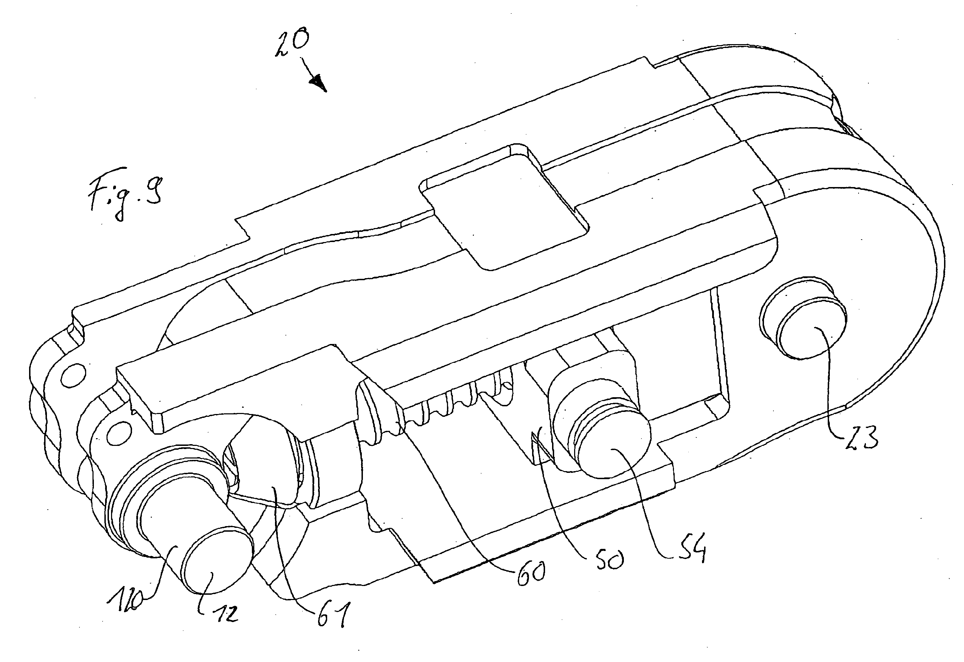

Die Figur 9 zeigt eine isolierte Darstellung des Medialgliedes 20 mit der montierten Spindel 60 und dem darauf angeordneten Kopplungselement 50. Um die Drehachse 12 herum ist eine kreisförmige Aussparung sichtbar, in die das Übertragungszahnrad 76 eingebracht werden kann. Das Übertragungszahnrad 76 wird auf einem Lagerabsatz 120 der Achse 12 gelagert und muss lediglich auf die Achse 12 aufgesteckt werden, um in Eingriff mit dem Kegelrad 61 zu gelangen. Die Achse 12 kann einstückig mit dem übrigen Rahmen des Medialglieds 20 ausgebildet sein, ebenso die distale Achse 23.

Zur Montage der Greifeinrichtung werden die in Längsrichtung geteilten Komponenten des Distalgliedes 30 und des Proximalgliedes 10 auf die Achsen 23, 12 aufgesteckt und fixiert, ebenso werden die seitlichen Hebel 40 in die Aufnahmeausnehmungen 14, 34 eingeführt, zusammen mit dem Einführen der Achse 54 in die Durchgangsöffnung 44. Dadurch ist die mechanische Kopplung fertiggestellt. Die Ausgestaltung ermöglicht eine einfache Montage durch eine gleich bleibende Fügerichtung der einzelnen Komponenten, die lediglich seitlich aufgesteckt und fixiert werden müssen.

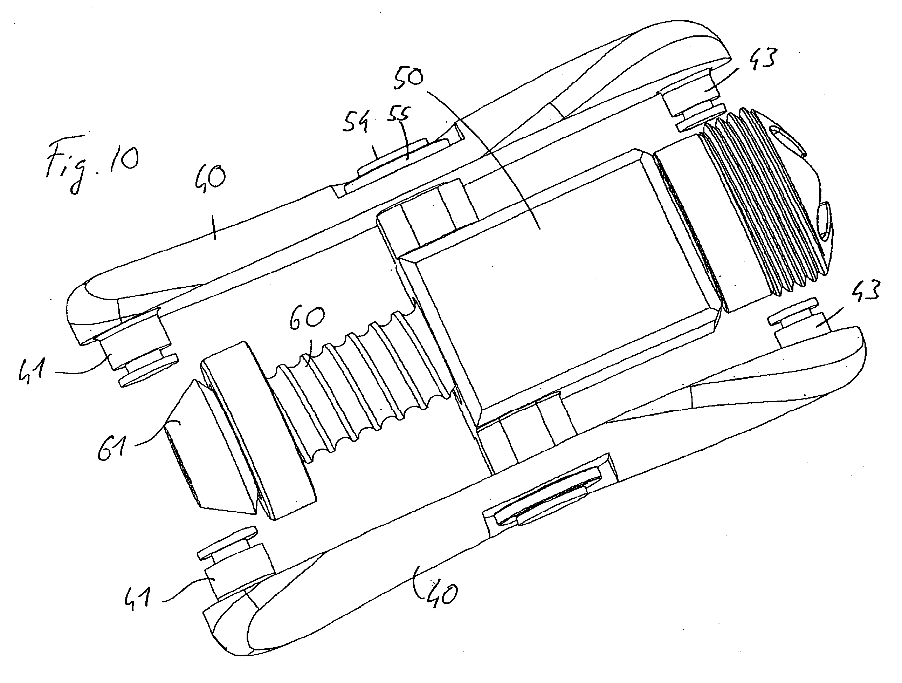

In der Figur 10 ist die Kraftübertragungsmechanik in einer Draufsicht gezeigt. Deutlich sind die beidseitig des Kopplungselementes 50, das als Spindelmutter ausgebildet ist, angeordneten Hebel 40 und die einander gegenüberliegenden Zapfen 41, 43 zu erkennen. An dem distalen Ende der Spindel 60 ist eine Lagerung der Spindel 60 sowie eine Sperreinrichtung zu erkennen, an dem proximalen Ende der Spindel 60 ist die Lagerung und das Kegelrad 61 angeordnet.

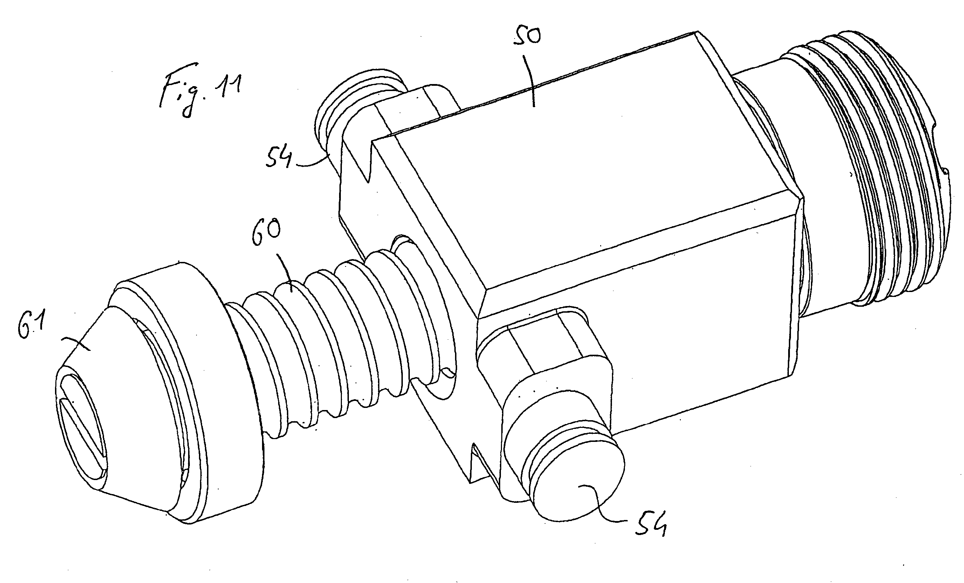

Die Figur 11 zeigt eine Detailansicht der Spindel 60 mit dem Kopplungselement 50 und den an dem Kopplungselement 50 angeformten Achsen 54. Das Kegelrad 61 ist an der Spindel 60 festgeschraubt, zwischen dem Kegelrad 61 und dem Bewegungsgewinde der Spindel 60 ist die proximale Lagerstätte angeordnet, die distale Lagerstätte ist an dem Kegelrad 61 abgewandten Ende der Spindel 60 zusammen mit der Sperreinrichtung angeordnet.

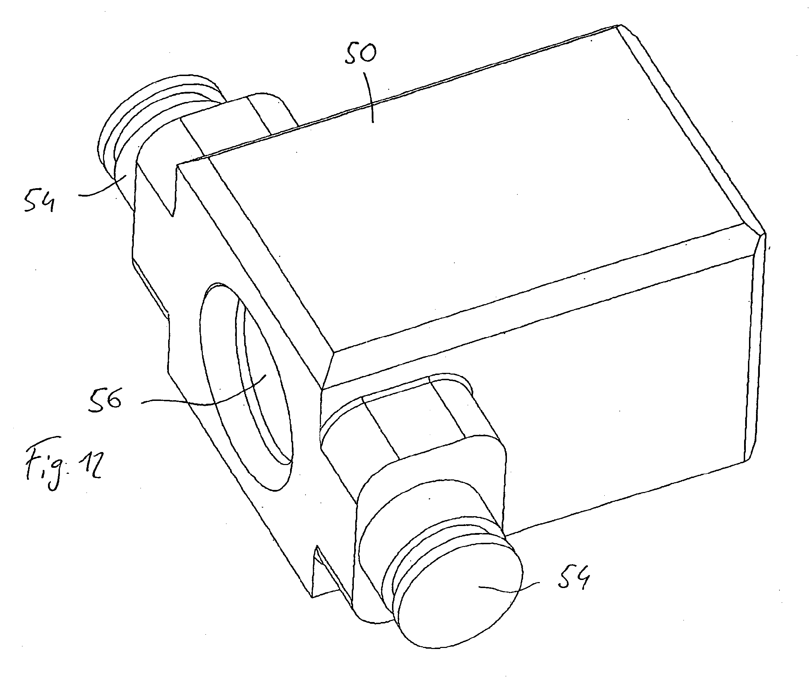

Die Figur 12 zeigt das Kopplungselement 50 in einer Detailansicht. Das Innengewinde 56 ist als Bewegungsgewinde ausgebildet, ebenfalls ist es möglich, dass ein Kugelumlaufgewinde vorhanden ist, bei dem in dem Bewegungsgewinde der Spindel 60 Kugeln umlaufen, die dann innerhalb des Kopplungselementes 50 umlaufen. Dadurch ist es möglich, mit einer sehr geringen Reibung die Greifeinrichtung 1 zu betreiben.

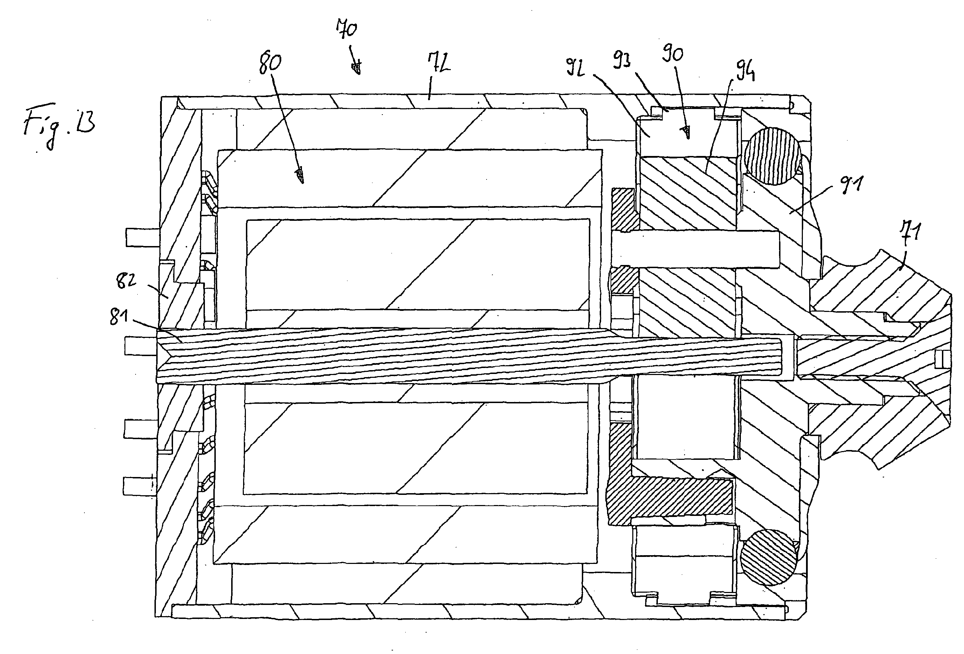

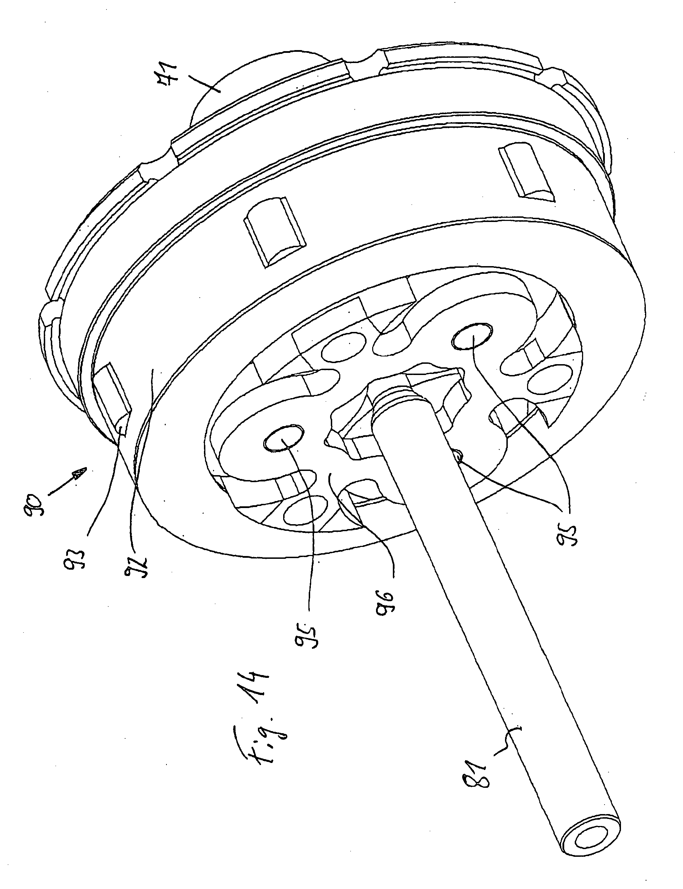

In der Figur 13 ist in einer Schnittdarstellung die Antriebseinheit 70, die in dem Proximalglied 10 angeordnet ist, dargestellt, die Antriebseinheit 70 sieht einen Motor 80 als Aktuator und ein Reibradgetriebe 90 vor, die in dem gemeinsamen Gehäuse 72 angeordnet sind. Der Motor 80 weist eine Abtriebswelle 81 auf, die proximal an einem Gleitlager oder Kugellager 82 und distal in dem Reibradgetriebe 90 gelagert ist. Das Reibradgetriebe 90 ist an einer Lagerscheibe 91 gelagert, auf der das Abtriebszahnrad 71 in Gestalt eines Kegelrades drehfest gelagert ist. Die vom Motor 80 aufgebrachte Antriebsleistung wird über das Reibradgetriebe 90 und die Lagerscheibe 91 auf das Abtriebszahnrad 71 übertragen. Das Reibradgetriebe 90 weist einen Laufring 92 auf, an dessen Außenseite Vorsprünge 93 ausgebildet sind, die in korrespondierende Ausnehmungen innerhalb des Gehäuses 72 eingreifen, so dass der Laufring 92 drehfest in dem Gehäuse 72 angeordnet ist. Innerhalb des Laufringes 92 laufen Reibräder 94 um, die auf Achsen 95 an einem Lagerkäfig 96 gelagert sind, was in der Figur 14 zu erkennen ist.

## Patentansprüche

1. Greifeinrichtung mit einem Proximalglied (10), einem Medialglied (20) und einem Distalglied (30), die verschwenkbar aneinander gelagert sind, und mit einem Aktuator (80), der mit einem verschieblich gelagerten Kopplungselement (50) gekoppelt ist, das Kopplungselement (50) ist zwischen dem Proximalglied (10) und dem Distalglied (30) angeordnet und mit dem Proximalglied (10) und dem Distalglied (30) kraftübertragend verbunden, **dadurch gekennzeichnet, dass** an dem Kopplungselement (50) zumindest ein Hebel (40) angeordnet ist, der sowohl mit dem Proximalglied (10) als auch mit dem Distalglied (30) verbunden ist und das Proximalglied (10) mit dem Distalglied (30) kinematisch koppelt.

2. Greifeinrichtung nach Anspruch 1, **dadurch gekennzeichnet, dass** der Aktuator (80) über eine Getriebeeinrichtung (71, 76, 61, 60, 50) mit dem Kopplungselement (50) gekoppelt ist.

3. Greifeinrichtung nach Anspruch 1 oder 2, **dadurch gekennzeichnet, dass** das Kopplungselement (50) als eine Spindelmutter ausgebildet ist.

4. Greifeinrichtung nach einem der voranstehenden Ansprüche, **dadurch gekennzeichnet, dass** das Kopplungselement (50) drehstarr mit dem Hebel (40) gekoppelt ist.

5. Greifeinrichtung nach einem der voranstehenden Ansprüche, **dadurch gekennzeichnet, dass** an dem Proximalglied (10) und dem Distalglied (30) Anlenkpunkte (14, 34) für den Hebel (40) vorgesehen sind, die auf einander gegenüberliegenden Seiten einer Verbindungsebene liegen, die durch die Schwenkachsen (12, 23) des Medialgliedes (20) und des Distalgliedes (30) verläuft.

6. Greifeinrichtung nach einem der voranstehenden Ansprüche, **dadurch gekennzeichnet, dass** der Hebel (40) einstückig ausgebildet ist.

7. Greifeinrichtung nach einem der Ansprüche 1 bis 5, **dadurch gekennzeichnet, dass** zwischen dem Hebel (40) und dem Kopplungselement (50) und/oder dem Proximalglied (10) und/oder dem Distalglied (30) ein elastisches Zusatzelement angeordnet ist.

8. Greifeinrichtung nach einem der voranstehenden Ansprüche, **dadurch gekennzeichnet, dass** der Hebel (40) beidseitig entlang einer Verschiebeachse des Kopplungselementes (50) an dem Medialglied (20) angeordnet ist.

9. Greifeinrichtung nach einem der voranstehenden Ansprüche, **dadurch gekennzeichnet, dass** der Aktuator (80) in dem Proximalglied (10) oder in dem Distalglied (30) angeordnet ist.

10. Greifeinrichtung nach einem der Ansprüche 2 bis 9, **dadurch gekennzeichnet, dass** die Getriebeeinrichtung ein Kegel- oder Kronenradgetriebe (71, 76, 61) und eine Spindel (60), ein Seil, eine biegsame Welle und/oder ein Kardangelenk aufweist.

11. Greifeinrichtung nach einem der voranstehenden Ansprüche, **dadurch gekennzeichnet, dass** der Aktuator (80) als Motor ausgebildet ist.

## Claims

1. Gripping device, comprising a proximal member (10), a medial member (20), and a distal member (30), which are mounted pivotably on each other, and comprising an actuator (80), which is coupled to a slidably mounted coupling element (50), the coupling element (50) being arranged between the proximal member (10) and the distal member (30) and being connected to the proximal member (10) and to the distal member (30) in a force-transmitting manner, **characterized in that** at least one lever (40) is arranged on the coupling element (50), which lever (40) is connected both to the proximal member (10) and also to the distal member (30) and kinematically couples the proximal member (10) to the distal member (30).

2. Gripping device as claimed in claim 1, **characterized in that** the actuator (80) is coupled to the coupling element (50) via a gear mechanism (71, 76, 61, 60, 50).

3. Gripping device as claimed in claim 1 or 2, **characterized in that** the coupling element (50) is designed as a spindle nut.

4. Gripping device as claimed in one of the preceding claims, **characterized in that** the coupling element (50) is coupled to the lever (40) in a rotationally rigid manner.

5. Gripping device as claimed in one of the preceding claims, **characterized in that** attachment points (14, 34) for the lever (40) are provided on the proximal member (10) and on the distal member (30), which attachment points (14, 34) lie on mutually opposite sides of a connecting plane that extends through the pivot axles (12, 23) of the medial member (20) and of the distal member (30).

6. Gripping device as claimed in one of the preceding claims, **characterized in that** the lever (40) is designed in one piece.

7. Gripping device as claimed in one of claims 1 through 5, **characterized in that** an elastic add-on element is arranged between the lever (40) and the coupling element (50) and/or the proximal member (10) and/or the distal member (30).

8. Gripping device as claimed in one of the preceding claims, **characterized in that** the lever (40) is arranged on the medial member (20) on both sides along a slide axis of the coupling element (50).

9. Gripping device as claimed in one of the preceding claims, **characterized in that** the actttator (80) is arranged in the proximal member (10) or in the distal member (30).

10. Gripping device as claimed in one of claims 2 through 9, **characterized in that** the gear mechanism has a bevel gear or crown wheel gear (71, 76, 61) and a spindle (60), a cable, a flexible shaft and/or a cardan joint.

11. Gripping device as claimed in one of the preceding claims, **characterized in that** the actuator (80) is designed as a motor.

## Revendications

1. Dispositif de préhension comprenant un organe proximal (10), un organe médial (20) et un organe distal (30), qui sont montés les uns sur les autres avec possibilité de pivotement, et comprenant un actionneur (80) qui est accouplé avec un élément de couplage (50) monté en translation, l'élément de couplage (50) est agencé entre l'organe proximal (10) et l'organe distal (30) et il est relié avec l'organe proximal (10) et avec l'organe distal (30) de manière à transmettre des forces, **caractérisé en ce qu'**au moins un levier (40) est agencé sur l'élément de couplage (50), levier qui est relié aussi bien avec l'organe proximal (10) qu'avec l'organe distal (30) et qui couple de manière cinématique l'organe proximal (10) avec l'organe distal (30).

2. Dispositif de préhension selon la revendication 1, **caractérisé en ce que** l'actionneur (80) est couplé à l'élément de couplage (50) via un mécanisme (71, 76, 61, 60, 50).

3. Dispositif de préhension selon la revendication 1 ou 2, **caractérisé en ce que** l'élément de couplage (50) est réalisé comme un écrou à broche.

4. Dispositif de préhension selon l'une des revendications précédentes, **caractérisé en ce que** l'élément de couplage (50) est couplé solidairement en rotation avec le levier (40).

5. Dispositif de préhension selon l'une des revendications précédentes, **caractérisé en ce qu'**il est prévu sur l'organe proximal (10) et sur l'organe distal (30) des points d'articulation (14, 34) pour le levier (40), qui se trouvent sur les côtés mutuellement opposés d'un plan de liaison qui passe par les axes de pivotement (12, 23) de l'organe médial (20) et de l'organe distal (30)

6. Dispositif de préhension selon l'une des revendications précédentes, **caractérisé en ce que** le levier (40) est réalisé d'une seule pièce.

7. Dispositif de préhension selon l'une des revendications 1 à 5, **caractérisé en ce qu'**un élément élastique additionnel est agencé entre le levier (40) et l'élément de couplage (50) et/ou l'organe proximal (10) et/ou l'organe distal (30).

8. Dispositif de préhension selon l'une des revendications précédentes, **caractérisé en ce que** le levier (40) est agencé des deux côtés le long d'un axe de translation de l'élément de couplage (50) sur l'organe médial (20).

9. Dispositif de préhension selon l'une des revendications précédentes, **caractérisé en ce que** l'actionneur (80) est agencé dans l'organe proximal (10) ou dans l'organe distal (30),

10. Dispositif de préhension selon l'une des revendications 2 à 9, **caractérisé en ce que** le mécanisme comprend un mécanisme à engrenages coniques ou un mécanisme à engrenages en couronne (71, 76, 61) et une broche (60), un câble, un arbre flexible et/ou un joint de cardan.

11. Dispositif de préhension selon l'une des revendications précédentes, **caractérisé en ce que** l'actionneur (80) est réalisé comme un moteur.
